Europäisches Patentamt

**European Patent Office**

Office européen des brevets

(19)

(11) Numéro de publication: **0 112 510**
**A1**

(12)

# DEMANDE DE BREVET EUROPEEN

(21) Numéro de dépôt: **83111867.4**

(22) Date de dépôt: **26.11.83**

(51) Int. Cl.³: **B 01 D 13/00,** B 01 D 31/00,
**A 61 M 1/03**

(30) Priorité: **30.11.82 BE 209609**

(43) Date de publication de la demande: **04.07.84**
**Bulletin 84/27**

(84) Etats contractants désignés: **AT BE CH DE FR GB IT LI
LU NL SE**

(71) Demandeur: **"Continental Beiro", Avenue Louise, 135,
B-1050 Bruxelles (BE)**

(72) Inventeur: **Beullens, Theophile G., Middelweg, 213,
B-3030 Leuven (BE)**

(74) Mandataire: **Callewaert, Jean et al, Bureau Gevers S.A.
rue de Livourne, 7 - Bte 1, B-1050 Bruxelles (BE)**

(54) **Procédé et dispositif pour régler la concentration de matières déterminées dans un liquide.**

(57) Procédé et dispositif pour régler la concentration de matières déterminées dans un liquide, suivant lequel on déplace ce liquide dans un compartiment (12) le long d'une des faces d'une paroi semi-perméable (24), sous une pression hydrodynamique déterminée (P1), on conduit ensuite une partie de ce liquide dans un compartiment 17 le long de la face opposée de cette paroi (24), suivant un sens sensiblement opposé (26) à celui du liquide se déplaçant le long de ladite première face, à une pression hydrodynamique (P1') inférieure à la pression susdite (P1), et on sépare l'autre partie de ce liquide par la conduite (28), le gradient de pression ainsi obtenu permettant de créer à travers cette paroi (24) simultanément une ultrafiltration et une osmose inverse augmentant la concentration (C1) en au moins une matière déterminée dans le liquide avant de passer le long de ladite face opposée.

EP 0 112 510 A1

- 1 -

0112510

"Procédé et dispositif pour régler la concentration
de matières déterminées dans un liquide."

La présente invention est relative à un procédé pour régler la concentration de matières déterminées dans un liquide, notamment pour séparer des matières d'un liquide et plus particulièrement pour traiter le sang de patients néphrétiques.

L'invention vise, d'une façon générale, un procédé pour purifier ou augmenter la concentration de solutions en une ou plusieurs substances bien déterminées, c'est-à-dire d'une manière sélective, dans des domaines très variés et ceci avec une consommation en énergie extrêmement réduite, ne nécissitant p.e. aucun échange de calories.

A cet égard, une application très particulière et très importante du procédé suivant l'invention réside dans le traitement du sang de patients, souffrant des reins, dans le but de supplier à la fonction rénale.

Les procédés utilisés généralement jusqu'à présent pour purifier le sang de tels patients sont basés sur la dialyse, qui consiste à extraire, à travers une membrane, par diffusion et osmose, des substances toxiques ainsi que de l'humidité superflue du corps, par la technique de l'ultrafiltration.

La dialyse utilise, à cet effet, un liquide de dialyse, appelé dialysat, avec lequel le sang entre en échange.

Cette technique présente différents inconvénients et extrait par exemple du sang une partie des éléments indispensables aux fonctions vitales.

Ainsi, un des buts essentiels de la présente invention consiste à remédier aux inconvénients des procédés connus pour la purification du sang et, de plus, comme déjà signalé ci-dessus, de présenter un procédé permettant de concentrer ou d'extraire d'une manière sélective des substances dissoutes dans des solutions avec un minimum de consommation en énergie.

A cet effet, suivant l'invention, on déplace le liquide à traiter le long d'une des faces d'une paroi semi-perméable, sous une pression hydrodynamique déterminée et suivant une direction sensiblement parallèle au plan de cette paroi, une partie de ce liquide étant ensuite conduite le long de la face opposée de cette paroi, suivant une direction sensiblement opposée à celle indiquée ci-dessus et à une pression hydrodynamique inférieure à la pression susdite, l'autre partie de ce liquide étant séparée, le gradient de pression ainsi obtenu permettant de créer à travers cette paroi simultanément une ultrafiltration et une osmose inverse augmentant la concentration en au moins une matière déterminée dissoute dans le liquide avant son passage le long de ladite face opposée.

Suivant une forme de réalisation préférée de l'objet de l'invention on utilise, comme liquide à traiter, du sang dont les globules et la fibrinogène ont préalablement été éliminées.

L'invention concerne également un dispositif pour régler la concentration de matières déterminées dans un liquide et notamment pour appliquer le procédé tel que décrit ci-dessus.

Ce dispositif est caractérisé par le fait qu'il comprend une enceinte allongée divisée suivant, sa direction longitudinale, en au moins deux compartiments par une paroi semi-perméable et présentant, d'une part, à une de ses extrémités une entrée vers le premier compartiment pour le liquide à traiter et une sortie du second compartiment et, d'autre part, à son extrémité opposée une sortie du premier compartiment pour ce liquide et une entrée vers le second compartiment, la sortie du premier compartiment étant liée à l'entrée du second compartiment de manière à obtenir ainsi un écoulement à contre-courant du liquide de part et d'autre de la paroi semi-perméable, des moyens étant prévus pour capter une partie du liquide au moins à la sortie du premier compartiment.

Suivant une forme de réalisation particulièrement avantageuse, le dispositif suivant l'invention est constitué d'un rein artificiel.

D'autres détails et particularités de l'invention ressortiront de la description donnée ci-après, en titre d'exemples non limitatifs, de quelques formes de réalisation particulières du procédé et du dispositif suivant l'invention, avec référence aux dessins annexés.

La figure 1 est une vue schématique en coupe longitudinale d'un dispositif suivant une première forme de réalisation de l'objet de l'invention pouvant faire office de rein artificiel.

La figure 2 est une vue schématique d'une coupe longitudinale, à plus grande échelle, d'une partie du dispositif suivant la figure 1.

La figure 3 est une vue schématique en coupe longitudinale d'une deuxième forme de réalisation du dispositif suivant l'invention.

La figure 4 est une vue schématique en coupe longitudinale d'une troisième forme de réalisation du dispositif suivant l'invention.

Dans les différentes figures, les mêmes chiffres de référence concernent les mêmes éléments ou des éléments analogues.

D'une façon générale, le procédé suivant l'invention est destiné à régler la concentration de matières solubles dans un liquide, et plus particulièrement pour séparer d'une manière sélective certaines matières spécifiques dissoutes dans un liquide.

Suivant ce procédé on fait couler ce liquide le long d'une des faces d'une paroi semi-perméable, à un débit et sous une pression hydrodynamique prédéterminés, suivant une direction sensiblement parallèle au plan de cette paroi.

Ensuite, on capte une partie de ce liquide et on conduit l'autre partie le long de la face opposée de cette paroi suivant une direction sensiblement opposée à celle indiquée ci-dessus et à une pression hydrodynamique inférieure à la pression susdite.

Par suite de la différence de pression qui existe ainsi de part et d'autre de la paroi semi-perméable on obtient simultanément une ultrafiltration et une osmose inverse, ayant comme résultat une augmentation graduelle de la concentration d'au moins une des matières déterminées dans le liquide pendant son déplacement le long de la première face de la paroi semi-perméable avant son passage à ladite face opposée.

Ceci a donc comme conséquence qu'une concentration maximale est obtenue au moment où l'on capte et sépare une partie du liquide et que l'on conduit la partie restante le long de la face opposée de la paroi semi-perméable.

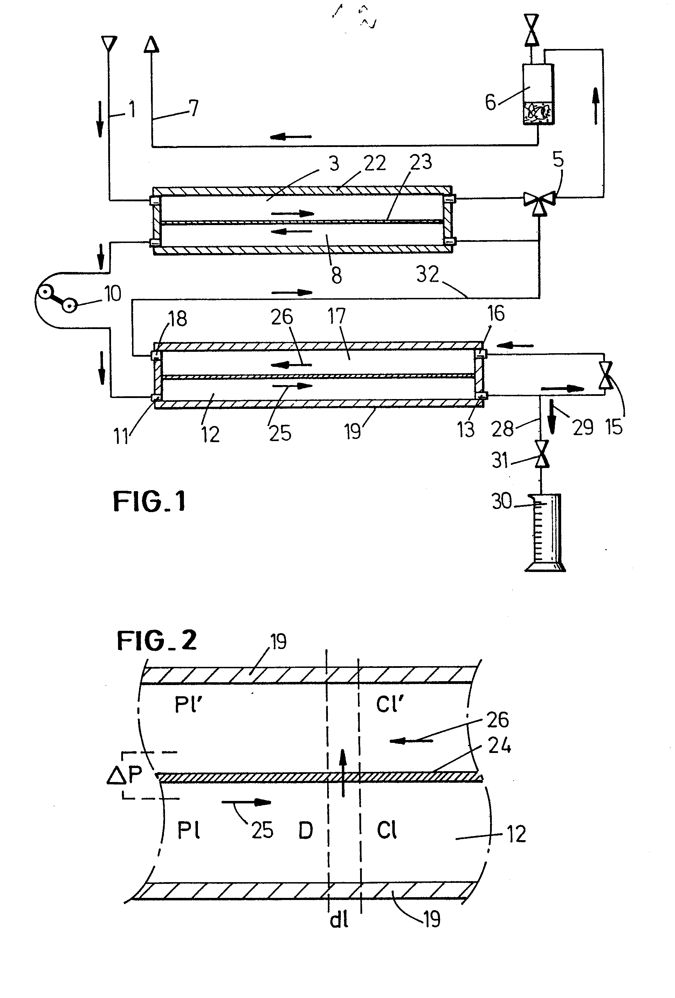

Les différentes caractéristiques du procédé suivant l'invention seront illustrées à la lumière de la forme de réalisation du dispositif, suivant l'invention, représentée aux figures 1 et 2.

Ce dispositif, qui est particulièrement approprié pour servir de rein artificiel, comprend essentiellement une boîte fermée 19, ayant la forme d'un parallélépipède rectangle relativement plat et allongé délimitant une enceinte divisée, suivant sa direction longitudinale, en deux compartiments 12 et 17 par une paroi semi-perméable 24, constituée par exemple d'une membrane.

Le liquide à traiter est introduit dans le compartiment 12 à une de ses extrémités par l'entrée 11 et se déplace dans le sens indiqué par la flèche 25 vers la sortie 13, située à l'extrémité opposée de ce compartiment.

Ensuite, une partie du liquide sortant du compartiment 12 est captée et séparée par une conduite de déviation 28, comme indiqué par la flèche 29, pour p.e. être collectée et stockée dans un récipient 30. La partie restante, qui présente de loin la quantité la plus importante, est guidée vers l'entrée 16 du second compartiment 17 à travers une vanne d'étranglement 15.

Le débit relatif de la quantité de liquide séparée est réglée par une vanne 31 prévue sur la conduite 28.

Cette partie restante du liquide se déplace alors à travers le second compartiment 17 le long de la face opposée de la paroi semi-perméable 24, comme indiquée par la flèche 26, à contre-courant par rapport au liquide se déplaçant dans le compartiment 12, pour quitter le compartiment 17 par la sortie 18.

Afin d'assurer une pression hydrodynamique suffisante du liquide dans le premier compartiment 12, le liquide peut y être introduit au moyen d'une pompe 10, qui, suivant l'application envisagée, peut p.e. être une pompe péristaltique.

Par ailleurs, notamment en fonction de la nature de la paroi semi-perméable 24 ou du liquide à traiter, il peut être nécessaire de soumettre ce dernier à un prétraitement telle qu'une filtration pour retenir toutes les particules non dissoutes.

Ceci est e.a. le cas pour le traitement du sang d'un patient souffrant d'une insuffisance dans la fonction rénale.

Il est important pour une telle application de soumettre le sang à une préfiltration, de manière à éliminer les globules et la fibrinogène et éviter aussi la coagulation du sang.

Plus particulièrement, il faut, pour obtenir un rendement maximum, que le liquide introduit dans le premier compartiment 12 soit constitué d'un sérum exempt de particules non dissoutes, telles que des globules et des colloïdes sous forme de suspensions et émulsions, mais dont, pour le restant, la composition correspond à celle du sang normal.

Ainsi, dans la forme de réalisation, montrée à la figure 1, on prévoit un filtre 22 divisé en deux zones 3 et 8 par une paroi perméable 23, telle qu'une membrane.

Ce filtre 22 peut, comme l'appareil à contre-courant 19, être constitué d'une enveloppe ayant la forme d'un parallélépipède rectangulaire plat et allongé.

Du sang artériël provenant du patient est introduit en continu par une des extrémités de la zone 3 du filtre 22, comme indiqué par la conduite 1. Ce sang circule le long de la paroi 23 et, le sang non filtré ainsi que les particules du sang filtré retenues par cette paroi sont alors évacués par la sortie 4 à l'extrémité opposée de cette zone 3 pour être reinjectés directement dans le sang veineux du patient.

Ce sang est toutefois mélangé à nouveau au sérum ayant subi un traitement dans l'appareil 19 et amené par une conduite 32 vers une vanne mélangeuse 5, de sorte que le sang réinjecté est uniquement débarassé de la quantité souhaitée d'humidité et de substances nocives captées dans le récipient 30.

Pour éviter que de l'air soit entraîné, un capteur de bulles 6 est encore prévu sur la conduite de retour 7. Ce capteur peut éventuellement être remplacé ou complété par des moyens de contrôle et/ou des moyens pour administrer des médicaments.

Le filtrat ayant traversé la paroi filtrante 23, qui est donc constitué par le sérum précité, est collecté dans la zone 8, d'où il est pompé sous une pression déterminée par la pompe 10 dans le compartiment 1 pour suivre le circuit déjà décrit ci-dessus.

Les phénomènes d'ultrafiltration et d'osmose inverse, sur lesquels le procédé et le fonctionnement du dispositif, suivant l'invention, sont basés, ainsi que les paramètres qui influencent ces phénomènes seront examinés ci-après en considérant la figure 2 qui représente une portion de l'appareil à contre-courant 19 de la figure 1.

Le liquide est introduit dans le compartiment 12 à une pression Pl et, par la vanne 15, cette pression est réduite et ramenée à une pression Pl' dans le compartiment 17.

Par ultrafiltration un courant de liquide se produit du compartiment 12 à travers la paroi 23 vers le compartiment 17.

L'ultrafiltration pour une même paroi semi-perméable est proportionnelle à la surface de cette paroi et au gradient de pression $\Delta P = Pl - Pl'$.

Ceci permet d'établir l'équation suivante:

$$dQu = U \cdot B (Pl - Pl') \, dl \quad (1)$$

dans laquelle :

U : la constante d'ultrafiltration en $m^3/N \cdot sec$

B : la largeur de la paroi 23 perpendiculaire à la direction de déplacement du liquide en mètres.

Pl : la pression hydrodynamique dans le compartiment 12 en N/m² en un endroit l.

Pl' : la pression dans le compartiment 17 en N/m² à cet endroit l.

dl : la longueur infinitésimale de la paroi dans le sens de déplacement du liquide dans le compartiment 12.

dQu : le débit de l'ultrafiltration à travers la surface B.dl de la paroi en m³/sec.

Pour la simplicité il a été admis que la constante d'ultrafiltration U est indépendante du gradient de pression.

Si on admet qu'il existe une différence de concentration d'une matière déterminée dissoute dans les liquides circulant dans les compartiments 12 et 17, suivant "la loi de van 't Hoff", une pression osmotique se produit sur la paroi 24 qui est dirigée vers la solution ayant la plus grande concentration.

Ainsi, on peut établir l'équation suivante:

$$Po = R \cdot T (Cl - Cl') \qquad (2)$$

dans laquelle :

Po : la pression osmotique en N/m².

Cl : la concentration d'une matière déterminée dissoute dans le liquide circulant dans compartiment 12 à l'endroit l de la paroi semi-perméable 24.

Cl' : la concentration de cette matière dans le liquide circulant dans le compartiment 17 au même endroit l, (inférieure à la concentration Cl).

R : la constante des gaz parfaits en Nm/mol°K

T : la température absolue.

En pratique, la température peut être considérée comme étant sensiblement constante, p.e. de l'ordre de 37°C ou 310°K pour le sang.

Dans ces conditions $R \cdot T = W$

avec $W = 2577$ Nm/mol

$Po = W(Cl - Cl')$

Cette pression osmotique agissant en sens opposé à la pression hydrodynamique, celle-ci réduit l'ultrafiltration suivant l'équation:

$$d\ Qu = U \cdot B\ [(Pl - Pl')] - W\ (Cl - Cl')]\ dl$$

$$d\ Qu = U \cdot B\ (Pl - Pl')\ dl - U \cdot B \cdot W\ (Cl - Cl')\ dl \quad (3)$$

Simultanément, un diffusion de la matière dissoute a lieu à travers la paroi 24.

Si on considère que la constante de diffusion de la paroi est indépendante de la pression et des concentrations, il en résulte que la diffusion est proportionnelle au gradient de concentration et à la surface semi-perméable de la paroi 24. Ceci donne l'équation :

$$dM = \alpha \cdot B\ (Cl - Cl')\ dl \quad (4)$$

dans laquelle :

$dM$ : le transport de masse de la matière dissoute à travers la surface infinitésimale $B.dl$ en mol/sec de la paroi 23.

$B$ : la largueur de la surface en m.

$\alpha$ : la constante de diffusion en (mol/sec)/(mol/m³)/m² ou en m/sec.

$Cl - Cl'$ : le gradient de concentration à travers la paroi 23.

$dl$ : la longueur infinitésimale de la paroi en m.

L'ultrafiltration obtenue par le gradient de pression précité $\Delta$ P est freinée par la pression osmotique, tandis que cette dernière est réduite par la diffusion se produisant à travers la paroi 24.

Par le fait que dans un volume élémentaire $B.D.dl$ du compartiment 12 la quantité entrante diminuée de la quantité diffusée à travers la paroi 24 est égale à la quantité sortante augmentée par la quantité accumulée dans ce volume, on peut établir l'équation suivante :

$$Ql \cdot Cl - \alpha \cdot B\ (Cl - Cl')\ dl = Q_{l+dl}\ C_{l+dl} + B \cdot D \left(\frac{dCl}{dt}\right)\ dl \quad (5)$$

dans laquelle :

$Ql \cdot Cl$ : la quantité entrante en mol/sec

$\alpha \cdot B (Cl - Cl') \, dl$ : la quantité diffusée en mol/sec

$Q_{l + dl} \cdot C_{l + dl}$ : la quantité sortante à l'endroit $(l + dl)$ en mol/sec.

$B \cdot D \cdot dl \left(\dfrac{dCl}{dt}\right)$ : la quantité accumulée dans ledit volume élémentaire en m³ , D étant l'épaisseur perpendiculaire à la paroi 24, pour une variation de concentration $\left(\dfrac{dCl}{dtl}\right)$ en (mol/m³)/ sec.

L'équation d'équilibre du solvant peut s'écrire comme suit :

$$Ql - Qu = Q_{l + dl}$$

$$Ql - U \cdot B \, [(Pl - Pl') - W (Cl - Cl')] \, dl = Q_{l + dl}$$

$$Ql - U \cdot B (Pl - Pl') \, dl + U \cdot B \cdot W (Cl - Cl') \, dl = Q_{l + dl}$$

$Ql$ : le débit du solvant entrant en m³/sec.

$U \cdot B (Pl - Pl') \, dl$ : le débit d'ultrafiltration du solvant parsuite de la différence de pression $(Pl - Pl')$.

$U \cdot B \cdot W (Cl - Cl') \, dl$ : le débit du solvant sous l'effet de la pression osmotique parsuite du gradient de concentration $(Cl - Cl')$.

$Q_{l + dl}$ : le débit du solvant sortant à l'endroit $l + dl$ en m³/sec.

La création d'une différence de concentration à travers de la paroi 24 par le maintient dudit gradient de pression $\Delta$ P, avec comme résultat une ultrafiltration donnant à son tour naissance à une pression osmotique en sens opposé, est appelée osmose inverse. En d'autres mots, par suite de ce gradient de pression supérieur à celui de la pression osmotique, une osmose inverse se produit, c'est-à-dire du solvant du compartiment 12 est expulsé à travers la paroi semi-perméable vers le compartiment 17.

Des mêmes équations similaires que pour le compartiment 12 sont valables pour le compartiment 17.

On obtient ainsi :

$$Q_{(l + dl)'} \cdot C_{(l + dl)'} + \alpha \cdot B (Cl - Cl') \, dl = Ql' \cdot Cl' - BD \left(\dfrac{dCl'}{dt}\right) \, dl \qquad (7)$$

$$Q_{(l + dl)'} + U \cdot B (Pl - Pl') \, dl - U \cdot B \cdot W (Cl - Cl') \, dl = Ql' \qquad (8)$$

$Q_{(l + dl)'}$ : le débit d'entrée du compartiment 17 à l'endroit (l + dl) en m³/sec.

$C_{(l + dl)'}$ : la concentration d'entrée du compartiment 17 à l'endroit (l + dl) en mol/m³.

Ql' : le débit de sortie du compartiment 17 à l'endroit l en m³/sec.

Cl' : la concentration de sortie du compartiment 17 à l'endroit l en mol/m³

Les termes - BD $\left(\dfrac{dCl'}{dt}\right)$ de l'équation (7) et B . D $\left(\dfrac{dCl}{dt}\right)$ de l'équation (5) représentent les changements de concentration dans les volumes des deux compartiments 12 et 17.

En régime, c'est-à-dire lorsque le système est stabilisé, on peut admettre que $\dfrac{dCl}{dt} = 0$ et $\dfrac{dCl'}{dt} = 0$.

Les équations (5) et (7) deviennent par conséquent :

$$Ql . Cl - \alpha . B (Cl - Cl') dl = Q_{(l+ dl)} \cdot C_{(l + dl)}$$

$$Q_{(l + dl)'} \cdot C_{(l + dl)'} + \alpha . B (Cl - Cl') dl = Ql' Cl'$$

Par le fait que la sortie 13 du compartiment 12 est reliée à l'entrée 16 du compartiment 17 en passant par un étranglement obtenu par la vanne 15, le gradient de pression sera maintenu et l'effet de l'osmose inverse créera, à travers la paroi 24, un gradient de concentration constant. Ce gradient de concentration est local, ce qui signifie une augmentation de concentration à un endroit déterminé du compartiment 12 par rapport à la concentration locale au côté opposé de la paroi 24 dans le compartiment 17.

Par l'écoulement du liquide dans le sens de la flèche 25 dans le compartiment 12 cette concentration augmentera graduellement suivant ce sens et inversément on obtiendra une diminution graduelle de cette concentration dans le compartiment 17 dans le sens de la flèche 26.

Le résultat en est donc une augmentation de concentration importante à la sortie 13 du compartiment 12 et donc dans le liquide capté dans le récipient 30.

Par une substitution de l'équation (6) dans l'équation (5') et de l'équation (8) dans l'équation (7') et une intégration sur la longueur totale de la paroi 24, on constate que l'augmentation de concentration est une fonction du rapport de l'ultrafiltration à travers cette paroi et du débit à travers les compartiments 12 et 17.

Ainsi, on peut conclure que, dans un dispositif donné, pour un type déterminé de paroi semi-perméable, dont les constantes d'ultrafiltration et de diffusion pour une solution déterminée sont connues, l'augmentation totale de la concentration et la composition du point de vue poids moléculaire peuvent être réglées en contrôlant le débit d'entrée et le gradient de pression à travers la paroi semi-perméable.

Si la solution à traiter contient plusieurs substances dissoutes, de poids moléculaires différentes, on obtient pour chacune de ces substances une augmentation de concentration différente.

Etant donné que l'ultrafiltration est directement proportionnelle au gradient de pression appliqué, on a en principe intérêt à ce que ce gradient soit aussi élevé que possible.

Toutefois, pour des raisons techniques, notamment la résistance de la paroi semi-perméable 24, ce gradient de pression ne peut guère dépasser 18 bars, et est généralement de l'ordre de 2 bars pour les parois sémi-perméables pouvant convenir pour le traitement du sérum de sang.

La vitesse d'écoulement idéale du liquide le long de la paroi semi-perméable 24 est notamment fonction des caractéristiques constructives de l'appareil à contre-courant 19.

En effet, essentiellement deux paramètres doivent être pris en considération : le débit à traiter et la diffusion des substances dissoutes en sens opposé au sens du déplacement du liquide, qui est appelée "contre-diffusion".

Ces deux paramètres exigent donc une vitesse minimum. Cette dernière est cependant déterminée par l'épaisseur de la couche de liquide se déplaçant le long de la paroi 24 dans le compartiment et de l'effet de la "contre-diffusion".

L'épaisseur maximale admise de la couche de liquide doit être telle que le gradient de concentration, suivant l'épaisseur de cette couche, soit aussi réduit que possible.

Pour cette raison, il est par conséquent important que l'écoulement du liquide ait lieu en régime sensiblement turbulent.

Ainsi, étant donné que pour le traitement d'un débit déterminé de liquide , les inconvénients de la "contre-diffusion" augmentent avec l'épaisseur de la couche de liquide se déplaçant le long de la paroi semi-perméable, on a intérêt à réduire cette épaisseur autant que possible et d'utiliser des parois semi-perméables ayant une surface aussi grande que possible.

Pour le traitement du sang d'un patient à insuffisance rénale, par exemple, on vise à utiliser des couches de liquide ayant une épaisseur comprise entre 0,1 et 1 mm et de préférence de l'ordre de 0,5 mm, tandis que la surface de la paroi peut par exemple être de l'ordre de 3 à 5 m². Ceci permet de traiter des débits de sang de l'ordre de 0,2 à 1,5 l/min, et de préférence entre 0,5 et 1 l/min.

Pour augmenter la turbulence de l'écoulement du liquide et, par conséquent, l'homogénéité de la concentration des substances dans l'épaisseur de la couche du liquide, on pourrait, avantageusement faire usage de compartiments 12 et 17 présentant une surface intérieure rugueuse.

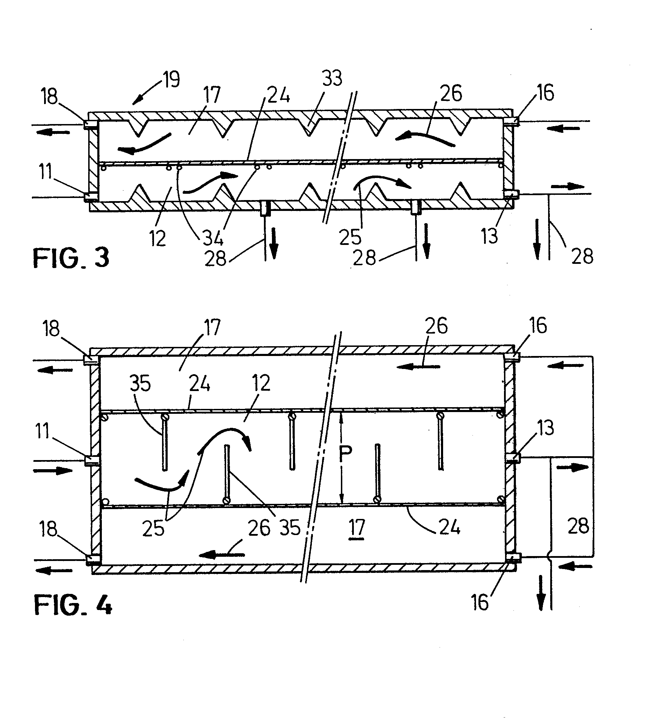

Les figures 3 et 4, concernent une deuxième et une troisième forme de réalisation du dispositif suivant l'invention qui sont particulièrement appropriées pour le traitement de débits relativement importants et qui pourraient aussi convenir pour le traitement et notamment la concentration de solutions à l'échelle industrielle, telle que la concentration de jus de fruits, de betteraves sucrières, etc.

Dans la forme de réalisation montrée à la figure 3 les compartiments 12 et 17 présentent, sur leurs surfaces intérieures, des nervures ou saillies 33 transversales au sens de l'écoulement du liquide, qui ont pour effet d'augmenter considérablement la turbulence et, de plus, par la division en cases successives ainsi obtenue, de réduire l'effet de la contre-diffusion, dont question ci-dessus.

De plus, des supports 34 sont prévus pour soutenir et maintenir la paroi semi-perméable 24 en place.

Par ailleurs, la possibilité existe de capter du liquide concentré en une série d'endroits successifs répartis dans le compartiment 12 suivant sa direction longitudinale.

Ainsi, la figure 3, présente à titre d'exemple deux conduits de déviations supplémentaires 28' et 28".

Ceci permet de récolter des fractions séparées de liquide qui présentent des compositions et concentrations différentes. Une telle forme de réalisation peut en quelque sorte être comparée avec une colonne de distillation fractionnée, toutefois avec l'importante différence qu'un apport de calories n'est pas nécessaire.

Dans la forme de réalisation suivant la figure 4, le compartiment 17 s'étend de part et d'autre du compartiment 12, ce dernier étant délimité par deux parois semi-perméables 24.

De plus, dans le compartiment 12, dont l'épaisseur D peut être relativement importante comparé à la forme de réalisation suivant la figure 1, des cloisons 35 sont prévus qui s'étendent alternativement à partir de la paroi semi-perméable supérieure et inférieure sur une certaine distance suivant la direction transversale du compartiment, de manière à créer un écoulement en chicanes du liquide, comme indiqué par les flèches 25.

Le liquide quittant le compartiment 12 par la sortie 13 se divise en deux courants sensiblement égaux et entre dans la zone supérieure et la zone inférieure du compartiment 17 par les entrées 16.

Pour traiter certaines solutions, par exemple des compositions plus ou moins complexes, on pourrait envisager de déplacer le liquide successivement le long de différents types de parois semi-perméables, notamment des parois à constante d'ultrafiltration croissante pour tenir compte du fait que les substances à bas poids moléculaire diffusent plus rapidement à travers la paroi 24 que celles de plus haut poids moléculaire.

D'une façon générale, on constate que des résultats satisfaisants sont obtenus pour traiter un nombre très variés de solutions et surtout du sang si les parois semi-perméables utilisées présentent une constante d'ultrafiltration relativement élevé, par exemple comprise entre (1ml/min) et (10 ml/min) / 100 mm $Hg/m^2$ et de préférence de l'ordre de (2 ml/min) / 100 mm $Hg/m^2$.

La constante de diffusion est un paramètre qui, d'une part, varie suivant la nature de la substance diffusante et, d'autre part, augmente en fonction de la vitesse d'écoulement du liquide le long de la paroi semi-perméable pour une même substance.

D'une façon générale, il a été constaté que des résultats satisfaisants sont obtenus si la constante de diffusion est relativement basse.

Par ailleurs, la vitesse de diffusion augmente avec le poids moléculaire.

Comme déjà signalé ci-dessus, en pratique, les deux paramètres à régler, pour un dispositif et une paroi semi-perméable donnés, sont la pression hydrodynamique à l'entrée du compartiment 12 et le débit du liquide introduit dans ce dernier.

Ainsi, si on désire concentrer essentiellement les molécules lourds, il y a lieu de travailler à des débits relativement petits. Si, de plus, on désire obtenir une concentration importante en ces molécules lourds la pression hydrodynamique doit être relativement grande.

Par ailleurs, si on désire concentrer également les substances à bas poids moléculaire, c'est-à-dire si on désire obtenir une solution concentrée dont la composition n'a pratiquement pas été modifiée, il y a lieu d'augmenter la vitesse, et, par conséquent, le débit dans le compartiment 12.

Outre de la solidité de la paroi semi-perméable, lors de l'application d'un pression au liquide envoyé dans le compartiment, il y a également lieu de tenir compte de la chute de pression dans le compartiment. D'une façon générale, pour un rein artificiel destiné à traiter du sérum de sang, on admet que la chute de pression maximale sur une longueur de 4 m ne peut guère dépasser 100 mm Hg.

Afin de permettre d'illustrer davantage le procédé et le dispositif suivant l'invention les caractéristiques essentielles appliquées au traitement de sérum de sang seront considérées plus en détail ci-après.

0112510

Le filtre 22 peut être un filtre courant utilisé pour la séparation de plasmas et est par exemple équipé d'une membrane à haute perméabilité en polyacrylnitrile. Un tel filtre permet d'obtenir, avec un gradient de pression relativement bas, par exemple de l'ordre de 50 mm Hg ou 6670 Pascals, un débit d'ultrafiltration d'au moins 100 ml/min, ou $1,6 \times 10^{-6}$ m³/sec. Uniquement les substances non dissoutes sont retenues, telles que les crytrocytes, leucocytes et trombocytes, de sorte que le filtrat recueilli dans la chambre 8 a une "osmolité" ou composition en substances dissoutes sensiblement analogue à celle du sang.

La perméabilité d'une telle membrane, appelée le "cut off", est généralement limitée à des substances ayant un poids moléculaire de l'ordre de 100.000, de sorte que la fibrinogène par exemple n'apparaît pas dans le filtrat. Il n'y a donc aucun risque de coagulation du filtrat ou sérum obtenu dans la chambre 8 du filtre 22.

La pompe peut être par exemple une pompe péristaltique utilisée en cardiochirurgie en dialyse, avec l'exception cependant que des pressions jusqu'à 2 bars sont atteintes, de sorte que le gradient de pression, qui en résulte, sur la paroi semi-perméable 24 dans l'appareil à contre-courant 19 permet d'entretenir un pression osmotique de l'ordre de 80 mol/m³.

Comme déjà signalé ci-dessus, les couches de liquide dans les compartiments 12 et 17 doivent de préférence être relativement minces, alors que la longueur totale de la paroi semi-perméable 24 doit être relativement importante, par exemple de l'ordre de 4000 mm. pour une largeur de l'ordre de 500 mm., de sorte que la surface utile est de 2 m² pour un volume total de $4 \times 10^{-3}$ m³, si l'épaisseur de la couche est de 1 mm.

Comme il résulte également déjà des considérations développées ci-dessus, si des précautions spéciales ne sont pas prises, il est indispensable de maintenir une couche mince du liquide se déplaçant le long de la paroi semi-perméable pour éviter un gradient de densité dans le sens de l'épaisseur de la couche.

Pour un débit de sérum de 100 ml/min, on obtient ainsi une vitesse d'écoulement de 20 cm/min qui, suivant la nature des moyens qui ont été prévus pour réduire l'effet de ladite "contre-diffusion", est généralement nécessaire pour obtenir un rendement suffisant.

Lors du démarrage du dispositif, les chambres 3 et 8 ainsi que les compartiments 12 et 17 sont remplis d'un sérum physiologique. Pour les dimensions données et pour le débit de 100 ml/min, le temps de passage à travers le dispositif est de l'ordre de 40 minutes, ce qui est le temps nécessaire pour que le dispositif soit en régime stable.

Etant donné qu'on vise à appliquer sur le sérum introduit dans le compartiment 12 une pression hydrodynamique aussi élevée que possible pour obtenir un rendement maximum, il est généralement nécessaire de prévoir les supports 33 pour la paroi semi-perméable 24.

Beaucoup de techniques sont connues à cet égard et sont appliquées dans divers types de reins artificiels, tels que le "screening" chez les types "coil" et "multipoint" chez les types "kill".

Une fonction supplémentaire de ces supports est qu'ils favorisent la turbulence dans le courrant de liquide.

Pour des raisons pratiques, les dimensions extérieures du dispositif suivant l'invention peuvent être réduites, p.e. en faisant usage d'un appareil à contre-courant 19 formé d'une double enveloppe souple enroulée sous forme de cylindre ou pliée en zigzag et à l'intérieure de laquelle se situe une paroi semi-perméable.

Il s'agit en fait d'une solution qui a déjà été envisagée dans certains reins artificiels connus.

Comme déjà indiqué ci-dessus, la paroi semi-perméable 24 doit présenter, de préférence une constante de diffusion élevée et une basse constante de diffusion pour le domaine moléculaire à concentrer du sérum.

Ces exigences, à première vue contradictoires, peuvent, par la technique actuelle des membranes, être réunies dans une même membrane, en appliquant par exemple lors de la fabrication de la membrane une coagulation plus rapide à la surface de celle-ci.

Ceci permet d'obtenir une composition hétérogène suivant l'épaisseur de la membrane et de régler le rapport entre l'ultrafiltration, qui est fonction de la résistance au passage, et la diffusion, qui est fonction de la section des pores à la surface de la membrane.

Généralement, la paroi ou membrane semi-perméable 24 est réalisée en polycarbonate, polysulfon et, en fait, en tous les matériaux utilisés dans les appareils de purification d'eau basés sur l'osmose inverse.

Etant donné que l'augmentation de la concentration dans le compartiment 12 est une fonction du rapport du débit du liquide dans ce compartiment et de l'ultrafiltration, il faut que la quantité collectée de liquide par la conduite de dérivation 28 par unité de temps soit relativement réduite par rapport à la quantité restante qui est introduite dans le compartiment 17.

Pour un patient à insuffisance rénale il y a généralement lieu de retirer 2000 ml d'humidité et de substances toxiques du sang.

Ces 2000 ml peuvent être extraits pendant une période de traitement d'environ 200 minutes, de sorte qu'une quantité de 10 ml/min est collectée dans le récipient 30 du dispositif montré à la figure 1.

Ceci signifie une réduction du débit dans le compartiment 17 de 10 %, ce qui provoquera une réduction de l'augmentation de la concentration à la sortie du compartiment 12.

Pour une constante d'ultrafiltration de la paroi 24 de (2ml/min) / (100 mm Hg/m²) ou 0,02 X $10^{-9}$ m³ / N sec on obtiendra une ultrafiltration d'environ 120 ml/min, ce qui correspond pratiquement au débit principal de l'appareil à contre-courant 19.

Ceci a donc comme résultat que, pour une paroi 24 à constante de diffusion relativement basse un accroissement de la concentration de l'ordre de vingt fois pour les petites molécules.

Pour un sérum qui présente à l'entrée 11 du compartiment 12 une concentration en urée de 2 g/l on obtient une augmentation de concentration jusqu'à 40 g/l et après le traitement un total de 80 g d'urée a été éliminé dans un volume de 2 l.

Ceci a comme conséquence que pour un patient, ayant un volume d'humidité de 50 l, on atteint une diminution de la concentration en urée de 1,6 g/l ou une concentration restante de 0,4 g/l.

Un raisonnement analogue est bien attendu applicable à toute autre substance dissoute dans le sérum. Il suffit de tenir compte des constantes de diffusion qui sont différentes pour chaque substance.

Il est bien entendu que l'invention n'est pas limitée aux formes de réalisation décrites et que bien des variantes peuvent être envisagées sans sortir du cadre de la présente invention. Ceci est notamment le cas pour les différentes applications possibles du procédé et de la construction du dispositif.

- 19 -

0112510

REVENDICATIONS :

1. Procédé pour régler la concentration de matières déterminées dans un liquide, notamment pour séparer des matières d'un liquide, caractérisé en ce qu'on déplace ce liquide le long d'une des faces d'une paroi semi-perméable, sous une pression hydrodynamique déterminée, en ce que l'on conduit ensuite une partie de ce liquide le long de la face opposée de cette paroi, suivant un sens sensiblement opposé à celui du liquide se déplaçant le long de ladite première face, à une pression hydrodynamique inférieure à la pression susdite, et en ce que, l'on sépare l'autre partie de ce liquide, le gradient de pression ainsi obtenu permettant de créer à travers cette paroi simultanément une ultrafiltration et une osmose inverse augmentant la concentration en au moins une matière déterminée dans le liquide avant de passer le long de ladite face opposée.

2. Procédé suivant la revendication 1, caractérisé en ce qu'on maintient le gradient de pression précité en étranglant le courant du fluide avant son passage le long de ladite face opposée de la paroi semi-perméable.

3. Procédé suivant l'une ou l'autre des revendications précédentes, caractérisé en ce qu'on maintient la pression hydrodynamique déterminée précitée par pompage.

4. Procédé suivant l'une quelconque des revendications précédentes, caractérisé en ce que le liquide se déplace le long de la paroi semi-perméable à une vitesse comprise entre 15 et 100 cm/min et de préférence entre 20 et 40 cm/min., de manière à éviter l'effet de la diffusion des substances dissoutes dans le sens opposé au sens de déplacement du liquide.

5. Procédé suivant l'une quelconque des revendications précédentes, caractérisé en ce qu'on déplace le liquide le long de la paroi semi-perméable en régime sensiblement turbulent.

6. Procédé suivant l'une quelconque des revendications précédentes, caractérisé en ce qu'on déplace le liquide le long de la paroi semi-perméable suivant une couche ayant une épaisseur comprise entre 0,2 et 1 mm., et de préférence de l'ordre de 0,5 mm.

7. Procédé suivant l'une quelconque des revendications précédentes, caractérisé en ce qu'on sépare ladite autre partie du liquide, à des endroits différents, répartis suivant la direction de déplacement du liquide le long de la première face de la paroi semi-perméable, permettant ainsi de récolter des fractions séparées de compositions et de concentrations différentes.

8. Procédé suivant l'une quelconque des revendications précédentes, caractérisé en ce qu'on déplace le liquide successivement le long de différents types de parois semi-perméables, notamment de parois à perméabilité croissante dans ce sens de déplacement du liquide.

9. Procédé suivant l'une quelconque des revendications précédentes, caractérisé en ce qu'on soumet le liquide à une filtration préalable au passage le long de la paroi semi-perméable précitée, de manière à retenir essentiellement les substances non dissoutes.

10. Procédé suivant l'une quelconque des revendications précédentes, caractérisé en ce qu'on utilise, comme liquide, une solution électrolytique colloïdale.

11. Procédé suivant la revendication 10, caractérisé en ce qu'on utilise, comme liquide, du sang dont les globules et la fibrinogène ont préalablement été séparées.

12. Procédé suivant la revendication 11, caractérisé en ce qu'on fait passer, d'une manière sensiblement continue, du sang artériel, provenant d'un patient, le long d'une paroi filtrante, de manière à ce qu'une partie de ce sang soit filtrée à travers cette paroi retenant essentiellement les globules et la fibrinogène, l'autre partie non filtrée de ce sang étant recyclée et reinjectée dans le patient, le filtrat ainsi obtenu étant amené le long de la paroi semi-perméable précitée, de manière à le concentrer, une partie de ce filtrat concentré étant séparée, l'autre partie étant ajoutée à ladite partie recyclée avant d'être réinjectée dans ce patient.

13. Dispositif pour régler la concentration de matières déterminées dans un liquide, notamment pour appliquer le procédé tel que décrit ci-dessus, ce dispositif étant caractérisé par le fait qu'il comprend une enceinte allongée divisée suivant sa direction longitudinale en au moins deux compartiments par au moins une paroi semi-perméable et présentant, d'une part, à une de ses extrémités une entrée du liquide vers le premier compartiment pour le liquide à traiter et une sortie du second compartiment, et, d'autre part, à son extrémité opposée une sortie du premier compartiment pour ce liquide et une entrée vers le second compartiment, la sortie du premier compartiment étant liée à l'entrée du second compartiment, de manière à obtenir un écoulement à contre-courant du liquide de part et d'autre de la paroi semi-perméable, des moyens étant prévus pour capter une partie du liquide avant d'entrer dans le second compartiment.

14. Dispositif suivant la revendication 13, caractérisé en ce que la paroi semi-perméable présente essentiellement une constante d'ultrafiltration élevée, notamment de (1ml/min) à (10 ml/min) / 100 mm Hg/m² et de préférence de l'ordre de (2 ml/min) / 100 mm Hg/m².

15. Dispositif suivant l'une ou l'autre des revendications 13 à 14, caractérisé en ce qu'au moins le premier compartiment comprend des moyens pour créer le long de la paroi semi-perméable un écoulement sensiblement turbulent du liquide à traiter.

16. Dispositif suivant la revendication 15, caractérisé en ce que ces moyens comprennent des saillies ou cloisons sur le passage du liquide à traiter, de manière à créer un courant sinueux.

17. Dispositif suivant l'une quelconque des revendications 13 à 16, caractérisé en ce qu'au moins le premier compartiment est divisé en une succession de cases communiquant entre-elles, et à travers desquelles passe le liquide, de manière à freiner l'effet de la diffusion dans ce liquide suivant le sens opposé au sens d'écoulement de ce dernier.

0112510

18. Dispositif suivant quelconque des revendications 13 à 17, caractérisé en ce qu'une vanne d'étranglement est prévue en amont de l'entrée vers le second compartiment, de manière à réduire la pression hydrodynamique du liquide entrant dans ce second compartiment.

19. Dispositif suivant l'une quelconque des revendications 13 à 18, caractérisé en ce que les moyens pour capter une partie du liquide avant d'entrer dans le second compartiment comprennent au moins une conduite de dérivation munie d'une vanne de réglage de débit.

20. Dispositif suivant la revendication 19, caractérisé en ce que des conduits de dérivation pour capter des liquides de différentes concentrations et compositions sont branchées sur le premier compartiment en des endroits décalés suivant la direction d'écoulement du liquide.

21. Dispositif suivant l'une quelconque des revendications 13 à 20, caractérisé en ce qu'il comprend une pompe en amont du premier compartiment permettant d'introduire le liquide dans ce dernier sous une pression hydrodynamique déterminée.

22. Dispositif suivant l'une quelconque des revendications 13 à 21, caractérisé en ce que l'enceinte précitée est précédée d'un filtre.

23. Dispositif suivant l'une quelconque des revendications 13 à 22, caractérisé en ce qu'il est constitué d'un rein artificiel.

24. Dispositif suivant l'une ou l'autre des revendications 22 et 23, caractérisé en ce que le filtre comprend une chambre dans laquelle est prévue une paroi perméable, qui, lorsque le liquide à traiter est constitué de sang, permet de retenir les globules et la fibrinogène, cette paroi divisant la chambre en deux zones, une zone dans laquelle circule le long de cette membrane du liquide non filtré qui est alors recyclé par une conduite de recirculation, l'autre zone permettant de récupérer le filtrat passant à travers la paroi, cette autre zone étant branchée, éventuellement par l'intermédiaire d'une pompe, sur l'entrée du premier compartiment de l'enceinte précitée, la sortie du second compartiment étant reliée à la conduite de recirculation, de manière à permettre de mélanger le liquide traité dans cette enceinte avec ladite partie non filtrée du liquide.

0112510

**FIG.1**

**FIG.2**

FIG. 3

FIG. 4

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| X | FR-A-2 120 745 (STATE OF ISRAEL)<br><br>* figure 1; revendications 1,3; page 6, lignes 11-27; page 9, lignes 13-31 * | 1,2,3, 10,13, 18,19 21 | B 01 D 13/00<br>B 01 D 31/00<br>A 61 M 1/03 |
| | --- | | |
| X | GB-A-2 053 024 (DOW CORNING CORP.)<br><br>* résumé; page 1, lignes 49-82; page 1, ligne 119 - page 2, ligne 36; page 2, ligne 122 - page 5, ligne 9; revendications 1,2,3; figures 1,3,4 * | 1,2,3, 4,13, 18,19 21 | |
| | --- | | DOMAINES TECHNIQUES RECHERCHES (Int. Cl. ³) |
| A | FR-A-2 021 564 (A.S. DE DANSKE SUKKERFABRIKKER)<br>* page 10, ligne 22 - page 11, ligne 3; figures 3,6; revendication 4 * | 5,15, 16 | |
| | --- | | B 01 D<br>A 61 H |
| A | EP-A-0 052 862 (COMP. GEN. D'ELECTRICITE)<br>* figures 1A,2; page 5, lignes 8-20 * | 5,6,15 ,16 | |
| | ---    -/- | | |

Le présent rapport de recherche a été établi pour toutes les revendications

| Lieu de la recherche<br>LA HAYE | Date d'achèvement de la recherche<br>27-03-1984 | Examinateur<br>HOORNAERT P.G.R.J. |
|---|---|---|

Office européen
des brevets

**RAPPORT DE RECHERCHE EUROPEENNE**

## DOCUMENTS CONSIDERES COMME PERTINENTS

Page 2

| Catégorie | Citation du document avec indication, en cas de besoin, des parties pertinentes | Revendication concernée | CLASSEMENT DE LA DEMANDE (Int. Cl. 3) |
|---|---|---|---|
| A | US-A-3 579 441 (HYDRONAUTICS INC.)<br><br>* figures 1A,3A; revendications 1,2,22,23; colonne 3, lignes 4-10; colonne 6, lignes 51-52 * | 3,6,9, 10,11, 12,21, 22,23, 24 | |
| | --- | | |
| A | EP-A-0 038 203 (KURARAY CO LTD.)<br><br>* résumé; figure 1 * | 3,9,10 ,11,12 ,21,22 ,23,24 | |
| | --- | | |
| A | PATENTS ABSTRACTS OF JAPAN, volume 2, no. 93, 29 juillet 1978, page 1661C78 & JP - A - 53/58974 (EBARA INFIRCO K.K.) * abrégé * | 8 | |
| | ----- | | DOMAINES TECHNIQUES RECHERCHES (Int. Cl. 3) |

Le présent rapport de recherche a été établi pour toutes les revendications

| Lieu de la recherche<br>LA HAYE | Date d'achèvement de la recherche<br>27-03-1984 | Examinateur<br>HOORNAERT P.G.R.J. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03.82